# EUROPEAN PATENT APPLICATION

(11) **EP 1 354 600 A1**
(43) Date of publication of application: **22.10.2003**
(21) Application number: 02008845.6
(22) Date of filing: 19.04.2002
(51) Int. Cl.: A61K 39/395, C12N 15/62, C12N 15/79, C12N 1/21, A61K 31/7088, A61P 35/00

(54) **Antibody combination useful for tumor therapy**

(71) Applicant: Affimed Therapeutics AG, 69120 Heidelberg (DE)
(72) Inventor: Kipriyanov, Sergey, 69126 Heidelberg (DE); Le Gall, Fabrice, 68535 Edingen-Neckarhausen (DE); Cochlovius, Björn, 1334 Haslum (NO); Little, Melvyn, 69151 Neckargemünd (DE)
(74) Representative: Schüssler, Andrea, Dr.

(57) **Abstract**

Disclosed is a combination of at least two antibodies, characterized by the following properties: (a) it comprises at least two different multivalent antibodies, each one having at least two specificities and being characterized by features: (b) + (d) for at least one of said two different multivalent antibodies, and (b) + (c) for at least one other of said two different multivalent antibodies, (b) is an antigen-binding domain specific to a tumor antigen, (c) is an antigen-binding domain specific to an antigen present on human T cells, and (d) is an antigen-binding domain specific to an antigen present on CD3-epsilon negative human effector cells. Also disclosed are polynucleotides encoding said antibodies as well as vectors comprising said polynucleotides, host cells transformed therewith and their use in the production of said antibodies. Finally, compositions, preferably pharmaceutical and diagnostic compositions, are disclosed comprising the above mentionned polynucleotides, antibodies or vectors. The pharmaceutical compositions are useful for immunotherapy, preferably against B cell malignancies, B cell-mediated autoimmune diseases and diseases associated with depletion of B cells.

## Description

The present invention relates to a combination of at least two antibodies, characterized by the following properties:(a) it comprises at least two different multivalent antibodies, each one having at least two specificities and being characterized by features (b) and (d) or (b) and (c) as defined below,( b) an antigen-binding domain specific to a tumor antigen, (c) an antigen-binding domain specific to an antigen present on human T cells, or (d) an antigen-binding domain specific to an antigen present on CD3-epsilon negative human effector cells. The present invention also relates to polynucleotides encoding said antibodies as well as vectors comprising said polynucleotides, host cells transformed therewith and their use in the production of said antibodies. Finally, the present invention relates to compositions, preferably pharmaceutical and diagnostic compositions, comprising the above mentioned polynucleotides, antibodies or vectors. The pharmaceutical compositions are useful for immunotherapy, preferably against B-cell malignancies, B-cell mediated autoimmune diseases and diseases associated with depletion of B-cells.

Non-Hodgkin's lymphoma (NHL) encompasses a heterogeneous group of hematological malignancies of B- and T-cell origin occurring in blood, lymph nodes and bone marrow, which frequently disseminate throughout the body. NHL is one of the few malignancies that have increased in frequency more than the increase in population, with approximately 53,000 new cases occurring annually in the United States. The most common forms of NHL are derived from the B cell lineage. While NHL can be treated with reasonable success at early and intermediate stages, the results of conventional chemotherapy and radiation in advanced stages remain disappointing. This holds particularly true for the prevalent low-grade lymphomas. A fairly large number of patients relapse and most remissions cannot be extended beyond the minimal residual disease.

Although chemotherapy and radiation therapy can induce clinical remissions in patients with NHL, this group of malignancies still remains a therapeutic challenge due to frequent lymphoma relapse and chemotherapy resistance. The malignant cells not destroyed by cytotoxic therapy appear to be responsible for treatment failure. These remaining tumor cells, referred to as minimal residual disease, are major targets for immunotherapeutic strategies, which include retargeting the cellular effector systems, such as T lymphocytes, NK cells or myeloid cells by bispecific antibodies (BsAbs). The BsAb makes a bridge between the tumor cell and the immune effector cell followed by triggering the cytotoxic responses that include perforin and granzyme release, Fas-mediated apoptosis and cytokine production. Since NHLs typically express one or more B cell markers, e.g. CD19 or CD20, these markers can be used to redirect effector cells towards malignant B cells. Although normal B cells will be also destroyed, they are repopulated from stem cells lacking the targeted Ags. To mediate redirected lysis, a BsAb must bind a target cell directly to a triggering molecule on the effector cell. The best-studied cytotoxic triggering receptors are multichain signaling complexes such as TCR/CD3 on T cells, FcγRIIIa (CD16) on NK cells, FcγRI (CD64) and FcαRI (CD89) expressed by monocytes, macrophages, and granulocytes. BsAbs directed to the TCR/CD3 complex have the potential to target all T cells, regardless of their natural MHC specificity. Thus far, different forms of the CD19 x CD3 BsAb have been generated and used in a number of in vitro and in vivo therapeutic studies. These BsAbs have been mainly produced using rodent hybrid hybridomas or by chemical cross-linking of two mAbs. However, the HAMA response and release of inflammatory cytokines are the major drawbacks of BsAb derived from rodent mAbs in clinical use. Recent advances in recombinant antibody technology have provided alternative methods for constructing and producing BsAb molecules. For example, CD19 x CD3 scFv-scFv tandems have been produced in mammalian cells. Alternatively, recombinant BsAbs can be formed by non-covalent association of two single chain fusion products consisting of the V_{H} and V_{L} domains of different specificity in an orientation preventing intramolecular pairing with the formation of a four-domain heterodimer diabody or an eight-domain homodimer tandem diabody. The two Ag-binding domains have been shown by crystallographic analysis to be on opposite sides of the diabody such that they are able to cross-link two cells. However, the thus far available BsAbs suffer from low cytotoxicity and the need of costimulatory agents in order to display satisfactory biological activity. However, the BsAbs directed to the TCR/CD3 complex alone suffer from low cytotoxicity because of the need of costimulatory agents to avoid T cell anergy and to trigger T cell cytotoxic activity.

Thus, the technical problem underlying the present invention was to provide means suitable for therapy of B-cell malignancies that overcome the disadvantages of the means of the prior art.

The solution to said technical problem is achieved by providing the embodiments characterized in the claims. The present invention is based on the observation that the generation of antibodies relying on retargeting of NK cells has a positive therapeutic effect, since, unlike T cells, FcR-bearing cellular mediators of innate immunity as e.g. NK cells (and monocytes, macrophages and granulocytes) tend to exist in constitutively activated states and do not need additional (pre-)stimulation. First, a bispecific diabody (BsDb) with reactivity against both human CD19 and FcγRIII (CD16) was constructed. Bacterially produced CD19 x CD16 BsDb specifically interacted with both CD19⁺ and CD16⁺ cells, and exhibited significantly higher apparent affinity and slower dissociation from the tumor cells than from effector cells. It was able to induce the specific lysis of tumor cells in the presence of isolated human NK cells or non-fractionated PBLs. The combination of the CD19 x CD16 BsDb with a previously described CD19 x CD3 BsDb and CD28 costimulation significantly increased the lytic potential of human PBLs. Treatment of SCID mice bearing an established Burkitt's lymphoma (5 mm in diameter) with human PBLs, CD19 x CD16 BsDb, CD19 x CD3 BsDb, and anti-CD28 mAb resulted in the complete elimination of tumors in 80% of animals. In contrast, mice receiving human PBLs in combination with either diabody alone showed only a partial tumor regression. These data clearly demonstrate the synergistic effect of small recombinant bispecific molecules recruiting different populations of human effector cells to the same tumor target and, thus, their usefulness for therapy.

Furthermore, monoclonal BsAbs used so far for therapy have immunoglobulin constant domains, which are responsible for undesired immune reactions (HAMA response). Thus, a preferred embodiment of the antibodies of the present invention are BsAb which only comprise the variable immunoglobulin domains, so called Fᵥ modules by means of which undesired immune responses can be avoided. Furthermore, they have a stability that makes it usable for therapeutic uses. The Fᵥ module is formed by association of the immunoglobulin heavy and light chain variable domains, V_{H} and V_{L}, respectively. The bispecific molecule, so called bispecific diabody (BsDb), can be formed by the noncovalent association of two single-chain fusion products, consisting of the V_{H} domain from one antibody connected by a short linker to the V_{L} domain of another antibody. Alternatively, recombinant BsAb, tandem diabody (Tandab) can be formed by homodimerization of single-chain molecules comprising four antibody variable domains (V_{H} and V_{L}) of two different specificities, in an orientation preventing intramolecular Fv formation.

Accordingly, the present invention relates to a combination of at least two antibodies, characterized by the following properties:
(a) it comprises at least two different multivalent antibodies, each one having at least two specificities and being characterized by features (b) and (d) or (b) and (c) as defined below;
(b) an antigen-binding domain specific to a tumor antigen;
(c) an antigen-binding domain specific to an antigen present on human T cells; or
(d) an antigen-binding domain specific to an antigen present on CD3-epsilon negative human effector cells.

Preferably, the antigen-binding domain (c) or (d) of the first antibody differs from the antigen binding domain (c) or (d) of the second antibody. Preferably, the first antibody is characterized by features (b) and (d) and the second antibody by features (b) and (c). For example, one antibody of the composition can be specific to both human CD19 on malignant B-cells and to human CD16 on cytotoxic NK-cells. Such antibody is capable of destroying CD19-positive tumor cells by recruitment of human NK-cells without any need for pre- and/or co-stimulation. This is in sharp contrast with any known bispecific molecules retargeting effector cells to CD19-positive target cells, such as CD19 x CD3 or CD19 x CD28 BsAbs. The independence from pre- and/or co-stimulation of effector cell population may substantially contribute to the therapeutic effect of such antibodies by augmentation of adverse side effects, such as cytokine release syndrome.

The antibodies of the composition of the present invention can be prepared by methods known to the person skilled in the art, e.g. by the following methods:
(a) Chemical coupling of antibodies or antibody fragments specific to, e.g., human CD19 and CD16, respectively, via heterobifunctional linkers.
(b) Fusion of hybridoma cell lines which are already available and which secrete monoclonal antibodies specific to, e.g., human CD19 and CD16, respectively.
(c) Transfection of the immunoglobulin light and heavy chain genes of at least two different specificities into murine myeloma cells or other eukaryotic expression systems and isolation of the polyvalent multispecific antibodies.

In a preferred embodiment, the tumor antigen recognized by the antibodies of the present invention is human CD19 which is - preferably - expressed on human B-cells.

In an alternativ preferred embodiment, the tumor antigen recognized by the antibodies of the present invention is human CD30 which is - preferably - expressed on Hodgkin's cells.

In a further preferred embodiment, the T-cell antigen recognized by the antibodies of the present invention is CD3, CD28 or CD5.

In a further preferred embodiment, the antigen present on CD3-epsilon negative human effector cells recognized by the antibodies of the present invention is CD16, CD64, CD32 or NKG-2D receptor.

It is also an object of the present invention to provide a combination of antibodies by means of which undesired immune responses, such as a HAMA response, can be avoided.

Thus, in a more preferred embodiment, the multivalent antibodies of the combination of the present invention are devoid of constant regions. Preferably, said antibodies are multimeric antibodies. This is, e.g., achieved by constructing a recombinant molecule, which consists only of non-immunogenic immunoglobulin variable (V_{H} and V_{L}) domains. This can also be achieved by using the antibody domains of human origin. Such antibodies can be prepared by methods known to the person skilled in the art, e.g. by the following methods:
(a) Construction of the multivalent multispecific single chain Fv-antibodies by combining the genes encoding at least four immunoglobulin variable V_{H} and V_{L} domains, either separated by peptide linkers or by no linkers, into a single genetic construct and expressing it in bacteria or other appropriate expression system.
(b) Non-covalent heterodimerization of two hybrid scFv fragments, each consisting of V_{H} and V_{L} domains of different specificity against a tumor antigen and an antigen present on CD3-epsilon negative human effector cells or an antigen present on human T-cells, either separated by peptide linkers or by no linkers, as a result of co-expression of corresponding genes or co-refolding of separately expressed corresponding precursors.
(c) Non-covalent homodimerization of single chain Fv-antibodies comprising at least four V_{H} and V_{L} domains of different specificity against a tumor antigen and an antigen present on CD3-epsilon negative human effector cells or an antigen present on human T-cells, either separated by peptide linkers or by no linkers, in an orientation preventing their intramolecular pairing.

In a preferred embodiment, the multivalent antibodies of the combination of the present invention are single chain Fv-antibodies comprising at least four immunoglobulin variable V_{H} and V_{L} domains, either separated by peptide linkers or by no linkers.

The term "Fv-antibody" as used herein relates to an antibody containing variable domains but not constant domains. The term "peptide linker" as used herein relates to any peptide capable of connecting two variable domains with its length depending on the kinds of variable domains to be connected. The peptide linker might contain any amino acid residue with the amino acid residues alanine, glycine, serine and proline being preferred. A preferred length of the peptide linker connecting two hybrid single chain Fv-antibodies is 0-30 amino acids with a length of 12 amino acid being more preferred. Preferably, the peptide linker connecting the variable V_{H} and V_{L} domains of a hybrid single chain Fv-antibody of the combination of the present invention comprises 0 to 12 amino acids, preferably 10 amino acids.

In a more preferred embodiment, the multivalent antibodies of the combination of the present invention are single chain Fv-antibodies comprising at least four immunoglobulin variable V_{H} and V_{L} domains, either separated by peptide linkers or by no linkers. Such an antibody can be generated, e.g., by combining the genes encoding at least four immunoglobulin variable V_{H} and V_{L} domains, either separated by peptide linkers or by no linkers, into a single genetic construct and expressing it in bacteria or other appropriate expression system.

In a further more preferred embodiment, the multivalent antibodies of the combination of the present invention are heterodimers of two hybrid single chain Fv-antibodies, each consisting of V_{H} and V_{L} domains of different specificity against a tumor antigen and an antigen present on CD3-epsilon negative human effector cells or an antigen present on human T-cells, either separated by peptide linkers or by no linkers. Such antibodies can be generated, e.g., by non-covalent heterodimerization of two hybrid single chain Fv-antibodies, each consisting of V_{H} and V_{L} domains of different specificity (against CD19 or CD16), either separated by peptide linkers or by no linkers, as a result of co-expression of corresponding genes or co-refolding of separately expressed corresponding precursors.

In an alternative further more preferred embodiment, the multivalent antibodies of the combination of the present invention are homodimers of single chain Fv-antibodies comprising at least four V_{H} and V_{L} domains of different specificity against a tumor antigen and an antigen present on CD3-epsilon negative human effector cells or an antigen present on human T cells, either separated by peptide linkers or by no linkers.

The present invention also relates to a combination of multivalent antibodies, wherein said antigen-binding domains mimic or correspond to V_{H} and V_{L} regions from a natural antibody. Preferably, said natural antibody is a monoclonal antibody, synthetic antibody, or humanized antibody.

In a further preferred embodiment, the combination of the present invention comprises a third antibody having an antigen-binding domain which is different from the antigen-binding domains of the first and second antibody. Particularly preferred is a combination comprising a first antibody which is a multivalent multimeric antibody specific to CD19 and CD16, a second antibody which is a multivalent multimeric antibody specific to CD19 and CD3, and, optionally, a third antibody which is specific for CD28.

In a further preferred embodiment, the variable V_{H} or V_{L} domains of the multivalent multimeric antibodies of the combination of the present invention are shortened by at least one amino acid residue at their N- and/or C-terminus.

The non-covalent binding of the multivalent multimeric antibody of the present invention can be strengthened by the introduction of at least one disulfide bridge between at least one pair of V-domains. This can be achieved by modifying the DNA sequences encoding the variable domains accordingly, i.e. by inserting in each of the DNA sequences encoding the two domains a codon encoding a cysteine residue or by replacing a codon by a codon encoding a cysteine residue.

Finally, the multivalent multimeric antibodies of the combination of the present invention can be further modified using conventional techniques known in the art, for example, by using amino acid deletion(s), insertion(s), substitution(s), addition(s), and/or recombination(s) and/or any other modification(s) known in the art either alone or in combination. Methods for introducing such modifications in the DNA sequence underlying the amino acid sequence of a variable domain or peptide linker are well known to the person skilled in the art; see, e.g., Sambrook, Molecular Cloning A Laboratory Manual, Cold Spring Harbor Laboratory (1989) N.Y.

At least one antibody of the combination of the present invention can comprise at least one further compound, e.g., a protein domain, said protein domain being linked by covalent or non-covalent bonds. The linkage can be based on genetic fusion according to the methods known in the art and described above or can be performed by, e.g., chemical cross-linking as described in, e.g., WO 94/04686. The additional domain present in the fusion protein comprising the antibody employed in accordance with the invention may preferably be linked by a flexible linker, advantageously a peptide linker, wherein said peptide linker comprises plural, hydrophilic, peptide-bonded amino acids of a length sufficient to span the distance between the C-terminal end of said further protein domain and the N-terminal end of the antibody or vice versa. The above described fusion protein may further comprise a cleavable linker or cleavage site for proteinases. Thus, e.g., at least one monomer of the antibody might be linked to a an effector molecule having a conformation suitable for biological activity or selective binding to a solid support, a biologically active substance (e.g. a cytokine or growth hormone), a chemical agent (e.g. doxorubicin, cyclosporin), a peptide (e.g. α-amanitin), a protein (e.g. granzyme A and B) or a drug.

Another object of the present invention is a process for the preparation of a combination of multimeric antibodies as described above, wherein (a) DNA sequences encoding the peptid linkers are ligated with the DNA sequences encoding the variable domains such that the peptide linkers connect the variable domains resulting in the formation of a DNA sequence encoding a monomer of the multivalent multimeric Fv-antibody, (b) the DNA sequences encoding the various monomers are expressed in a suitable expression system and (c) the antibodies are combined. The various steps of this process can be carried according to standard methods, e.g. methods described in Sambrook et al., or described in the Examples, below.

The present invention also relates to polynucleotides encoding the antibodies of the combination of the present invention and vectors, preferably expression vectors containing said polynucleotides.

A variety of expression vector/host systems may be utilized to contain and express sequences encoding the antibodies. These include, but are not limited to, microorganisms such as bacteria transformed with recombinant bacteriophage, plasmid, or cosmid DNA expression vectors; yeast transformed with yeast expression vectors; insect cell systems infected with virus expression vectors (e.g., baculovirus); plant cell systems transformed with virus expression vectors (e.g., cauliflower mosaic virus, CaMV; tobacco mosaic virus, TMV) or with bacterial expression vectors (e.g., Ti or pBR322 plasmids); or animal cell systems.

The "control elements" or "regulatory sequences" are those non-translated regions of the vector-enhancers, promoters, 5' and 3' untranslated regions which interact with host cellular proteins to carry out transcription and translation. Such elements may vary in their strength and specificity. Depending on the vector system and host utilized, any number of suitable transcription and translation elements, including constitutive and inducible promoters, may be used. For example, when cloning in bacterial systems, inducible promoters such as the hybrid lacZ promoter of the Bluescript.RTM. phagemid (Stratagene, LaJolla, Calif.) or pSport1.TM. plasmid (Gibco BRL) and the like may be used. The baculovirus polyhedrin promoter may be used in insect cells. Promoters or enhancers derived from the genomes of plant cells (e.g., heat shock, RUBISCO; and storage protein genes) or from plant viruses (e.g., viral promoters or leader sequences) may be cloned into the vector. In mammalian cell systems, promoters from mammalian genes or from mammalian viruses are preferable. If it is necessary to generate a cell line that contains multiple copies of the sequence encoding the multivalent multimeric antibody, vectors based on SV40 or EBV may be used with an appropriate selectable marker.

In bacterial systems, a number of expression vectors may be selected depending upon the use intended for the antibodies of the combination of the present invention. Vectors suitable for use in the present invention include, but are not limited to the pSKK expression vector for expression in bacteria.

In the yeast, Saccharomyces cerevisiae, a number of vectors containing constitutive or inducible promoters such as alpha factor, alcohol oxidase, and PGH may be used. For reviews, see Grant et al. (1987) Methods Enzymol. 153:516-544.

In cases where plant expression vectors are used, the expression of sequences encoding the antibodies of the combination of the present invention may be driven by any of a number of promoters. For example, viral promoters such as the 35S and 19S promoters of CaMV may be used alone or in combination with the omega leader sequence from TMV (Takamatsu, N. (1987) EMBO J. 6:307-311). Alternatively, plant promoters such as the small subunit of RUBISCO or heat shock promoters may be used (Coruzzi, G. et al. (1984) EMBO J. 3:1671-1680; Broglie, R. et al. (1984) Science 224:838-843; and Winter, J. et al. (1991) Results Probl. Cell Differ. 17:85-105). These constructs can be introduced into plant cells by direct DNA transformation or pathogen-mediated transfection. Such techniques are described in a number of generally available reviews (see, for example, Hobbs, S. and Murry, L. E. in McGraw Hill Yearbook of Science and Technology (1992) McGraw Hill, New York, N.Y.; pp. 191-196.

An insect system may also be used to express the antibodies of the combination of the present invention. For example, in one such system, Autographa californica nuclear polyhedrosis virus (AcNPV) is used as a vector to express foreign genes in Spodoptera frugiperda cells or in Trichoplusia larvae. The sequences encoding the antibody may be cloned into a non-essential region of the virus, such as the polyhedrin gene, and placed under control of the polyhedrin promoter. Successful insertion of the gene encoding the multivalent multimeric antibody will render the polyhedrin gene inactive and produce recombinant virus lacking coat protein. The recombinant viruses may then be used to infect, for example, S. frugiperda cells or Trichoplusia larvae in which APOP may be expressed (Engelhard, E. K. et al. (1994) Proc. Nat. Acad. Sci. 91:3224-3227).

In mammalian host cells, a number of viral-based expression systems may be utilized. In cases where an adenovirus is used as an expression vector, sequences encoding the antibodies may be ligated into an adenovirus transcription/translation complex consisting of the late promoter and tripartite leader sequence. Insertion in a non-essential E1 or E3 region of the viral genome may be used to obtain a viable virus which is capable of expressing the multivalent multimeric antibody in infected host cells (Logan, J. and Shenk, T. (1984) Proc. Natl. Acad. Sci. 81:3655-3659). In addition, transcription enhancers, such as the Rous sarcoma virus (RSV) enhancer, may be used to increase expression in mammalian host cells.

Human artificial chromosomes (HACs) may also be employed to deliver larger fragments of DNA than can be contained and expressed in a plasmid. HACs of 6 to 10M are constructed and delivered via conventional delivery methods (liposomes, polycationic amino polymers, or vesicles) for therapeutic purposes.

Specific initiation signals may also be used to achieve more efficient translation of sequences encoding the antibodies. Such signals include the ATG initiation codon and adjacent sequences. In cases where sequences encoding the antibodies, its initiation codon, and upstream sequences are inserted into the appropriate expression vector, no additional transcriptional or translational control signals may be needed. However, in case where only coding sequence is inserted, exogenous translational control signals including the ATG initiation codon should be provided. Furthermore, the initiation codon should be in the correct reading frame to ensure translation of the entire insert. Exogenous translational elements and initiation codons may be of various origins, both natural and synthetic. The efficiency of expression may be enhanced by the inclusion of enhancers which are appropriate for the particular cell system which is used, such as those described in the literature (Scharf, D. et al. (1994) Results Probl. Cell Differ. 20:125-162).

In addition, a host cell strain may be chosen for its ability to modulate the expression of the inserted sequences or to process the expressed antibody chains in the desired fashion. Post-translational processing which cleaves a "prepro" form of the protein may also be used to facilitate correct insertion, folding and/or function. Different host cells which have specific cellular machinery and characteristic mechanisms for post-translational activities (e.g., CHO, HeLa, MDCK, HEK293, and W138), are available from the American Type Culture Collection (ATCC; Bethesda, Md.) and may be chosen to ensure the correct modification and processing of the foreign antibody chains.

For long-term, high-yield production of recombinant antibodies, stable expression is preferred. For example, cell lines which stably express the multivalent multimeric antibody may be transformed using expression vectors which may contain viral origins of replication and/or endogenous expression elements and a selectable marker gene on the same or on a separate vector. Following the introduction of the vector, cells may be allowed to grow for 1-2 days in an enriched media before they are switched to selective media. The purpose of the selectable marker is to confer resistance to selection, and its presence allows growth and recovery of cells which successfully express the introduced sequences. Resistant clones of stably transformed cells may be proliferated using tissue culture techniques appropriate to the cell type.

Any number of selection systems may be used to recover transformed cell lines. These include the herpes simplex virus thymidine kinase (Wigler, M. et al. (1977) Cell 11:223-32) and adenine phosphoribosyltransferase (Lowy, I. et al. (1980) Cell 22:817-23) genes which can be employed in tk.sup.- or aprt.sup.- cells, respectively. Also, antimetabolite, antibiotic or herbicide resistance can be used as the basis for selection; for example, dhfr which confers resistance to methotrexate (Wigler, M. et al. (1980) Proc. Natl. Acad. Sci. 77:3567-70); npt, which confers resistance to the aminoglycosides neomycin and G-418 (Colbere-Garapin, F. et al (1981) J. Mol. Biol. 150:1-14) and als or pat, which confer resistance to chlorsulfuron and phosphinotricin acetyltransferase, respectively (Murry, supra). Additional selectable genes have been described, for example, trpB, which allows cells to utilize indole in place of tryptophan, or hisD, which allows cells to utilize histinol in place of histidine (Hartman, S. C. and R. C. Mulligan (1988) Proc. Natl. Acad. Sci. 85:8047-51). Recently, the use of visible markers has gained popularity with such markers as anthocyanins, beta-glucuronidase and its substrate GUS, and luciferase and its substrate luciferin, being widely used not only to identify transformants, but also to quantify the amount of transient or stable protein expression attributable to a specific vector system (Rhodes, C. A. et al. (1995) Methods Mol. Biol. 55:121-131).

The present invention also relates to a composition containing the combination of antibodies as described above, polynucleotides encoding said antibodies or an expression vector. Preferably, said composition is a pharmaceutical composition preferably combined with a suitable pharmaceutical carrier or a diagnostic composition optionally further comprising suitable means for detection. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and can be administered to the subject at a suitable dose. Administration of the suitable compositions may be effected by different ways, e.g. by intravenous, intraperetoneal, subcutaneous, intramuscular, topical or intradermal administration. The route of administration, of course, depends on the nature of the disease, e.g. tumor, and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind of the disorder, general health and other drugs being administered concurrently.

Preferred medical uses of the combination of antibodies of the present invention described above are the treatment of a B-cell malignancy, preferably non-Hodgkin's lymphoma, a B-cell mediated autoimmune disease, a depletion of B-cells.

A further preferred medical use of the combination of antibodies of the present invention described above is the treatment of Hodgkin's disease.

Finally, the polynucleotides encoding the antibodies of the combination of the present invention are useful for gene therapy. Preferred recombinant vectors useful for gene therapy are viral vectors, e.g. adenovirus, herpes virus, vaccinia, or, more preferably, an RNA virus such as a Retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Further suitable vectors and methods for in vitro- or in vivo-gene therapy are described in the literature and are known to the persons skilled in the art; see, e.g., WO 94/29469 or WO 97/00957.

In order to achieve expression only in the target organ, e.g., a tumor to be treated, the polynucleotides encoding the antibodies of the combination of the present invention can be linked to a tissue specific promoter and used for gene therapy. Such promoters are well known to those skilled in the art (see e.g. Zimmermann et al., (1994) Neuron 12, 11-24; Vidal et al.; (1990) EMBO J. 9, 833-840; Mayford et al., (1995), Cell 81, 891-904; Pinkert et al., (1987) Genes & Dev. 1, 268-76).

### Brief description of the drawings

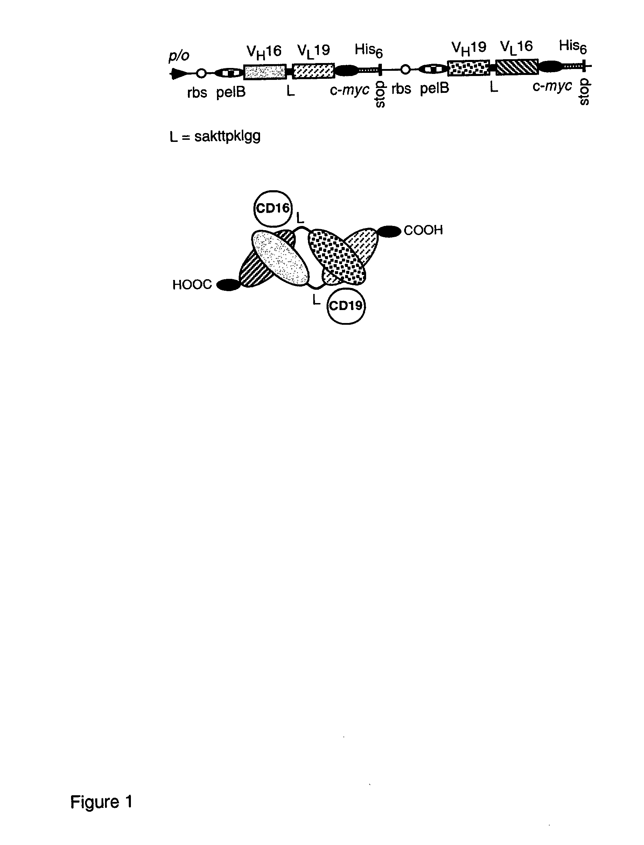

### FIGURE 1: Schematic representation of operon encoding CD19 x CD16 BsDb in plasmid pKID19x16 and protein model of BsDb

The locations of wild-type *lac* promoter/operator (*p*/*o*), ribosome binding sites (rbs), *pel*B leader sequences (pelB), c-*myc* epitope (c-*myc*), hexahistidine tag (His₆) and stop codon (stop) are indicated. The amino acid sequence of peptide linker between V_{H} and V_{L} domains is shown below the drawing. Carboxy termini (*COOH*), linkers (*L*), and CD16 and CD19 Ag-binding sites are indicated on the schematic model of BsDb.

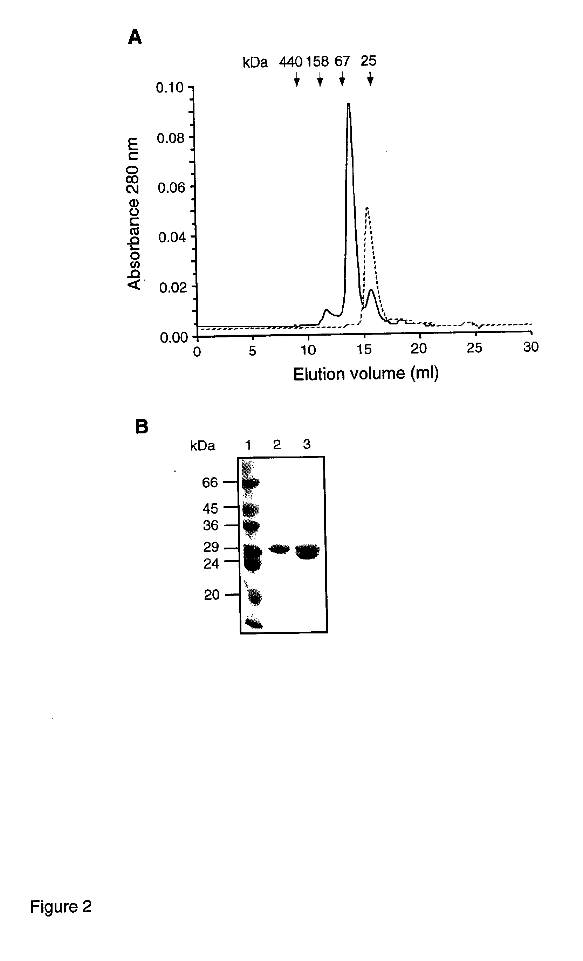

### FIGURE 2: Analyses of purified recombinant antibodies

***A,*** Elution profiles of CD19 x CD16 BsDb (―) and hybrid V_{H}19-V_{L}16 scFv (·····) from a calibrated Superdex 200 gel filtration column. ***B*,** 12% SDS-PAGE under reducing conditions. *Lane 1, M*ᵣ markers (kDa, *M*ᵣ in thousands); *Lane 2,* V_{H}19-V_{L}16 scFv; *Lane 3,* CD19 x CD16 BsDb. The gel was stained with Coomassie.

### FIGURE 3: Flow cytometric analysis of CD19 x CD16 BsDb binding to CD19⁺ JOK-1 cells (triangles) and CD16⁺ 293-CD16 cells (circles)

***A,*** Lineweaver-Burk analysis of fluorescence dependence on BsDb concentration. ***B*,** in vitro cell surface retention assay. Values are expressed as a percentage of initial mean fluorescence intensity.

### FIGURE 4: BsDb-mediated lysis of CD19⁺ Raji cells by human PBLs or NK cells at different E:T ratios

***A, B*,** Dose-dependent lysis of tumor cells by PBLs **(*A*)** or NK cells **(*B*)** in presence of CD19 x CD16 BsDb at concentration of 0.5, 1 and 5 µg/ml. C, Lysis of tumor cells by human PBLs mediated either by CD19 x CD16 BsDb or CD19 x CD3 BsDb alone at concentration of 5 µg/ml, or by combination of both BsDb at concentration 2.5 µg/ml. Experiments were done in triplicate; *bars* represent SDs of measurements for three different donors.

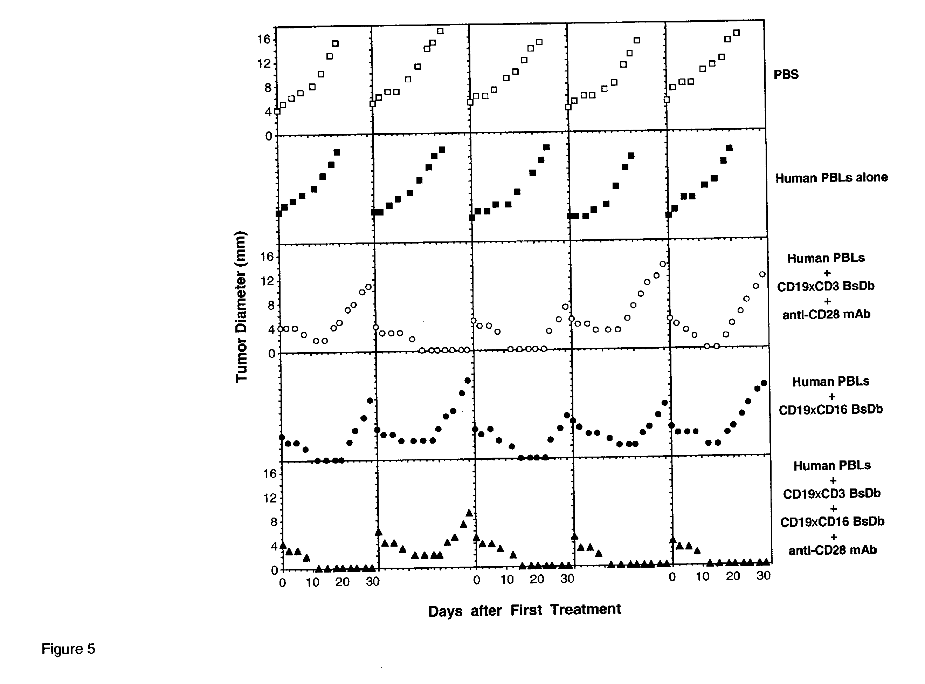

### FIGURE 5: Treatment of SCID mice bearing human Burkitt's lymphoma xenografts

The mice received PBS (open squares), preactivated human PBLs alone (closed squares), or preactivated human PBLs followed 4 h later by the administration of CD19 x CD3 BsDb plus mAb 15E8 (open circles), CD19 x CD16 BsDb alone (closed circles) or CD19 x CD16 BsDb in combination with CD19 x CD3 BsDb and mAb 15E8 (closed triangles). Tumor size was measured every second day. Tumor growth curves of individual animals till 30 day of experiment are presented.

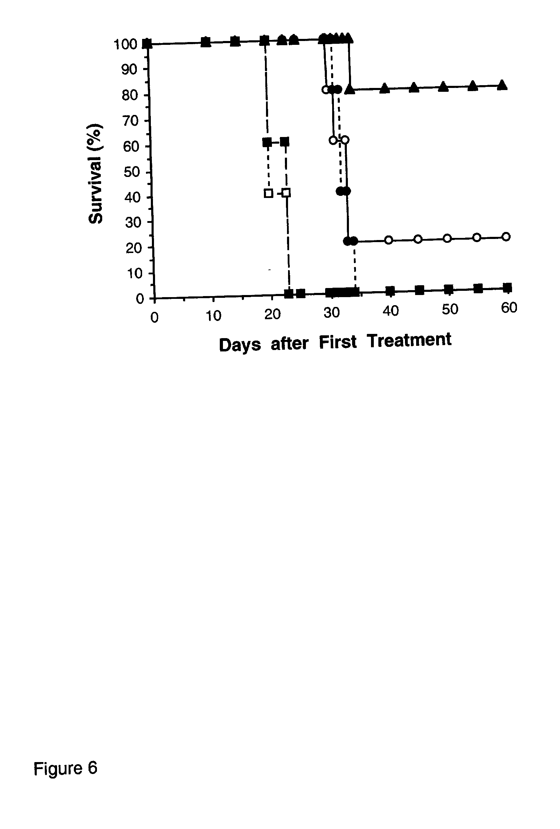

### FIGURE 6: Survival of SCID mice bearing human Burkitt's lymphoma xenografts

The mice received PBS (open squares); or preactivated human PBLs alone (closed squares); or preactivated human PBLs followed 4 h later by the administration of CD19 x CD3 BsDb plus mAb 15E8 (open circles), CD19 x CD16 BsDb alone (closed circles), or CD19 x CD16 BsDb in combination with CD19 x CD3 BsDb and mAb 15E8 (closed triangles).

The following Examples illustrate the invention.

### Example 1: General methods

### (A) Materials and cell lines

ECD of human FcgRIII (CD16) was a kind gift of Dr. G. P. Adams, Fox Chase Cancer Center, Philadelphia, PA. The human embryonic kidney (HEK) 293 cells stably transfected with human CD16B cDNA (293-CD16) were kindly provided by Dr. R. E. Schmidt, Department of Clinical Immunology, Medical School Hannover, Hannover, Germany. Human CD19⁺ cell lines JOK-1 and Raji, as well as CD3⁺ cell line Jurkat were from the cell line collection of the German Cancer Research Center (DKFZ). CD19 x CD3 BsDb was previously described (Kipriyanov et al., In. J. Cancer 77 (1998), 763; Cochlovius et al., J. Immunol. 165 (2000), 888.

### (B) Construction and production of CD19 x CD16 BsDb

The genes coding for V_{H}16-V_{L}19 and V_{H}19-V_{L}16 hybrid scFvs were constructed by exchange of the anti-CD3 V_{H} and V_{L} genes in plasmids pHOG3-19 and pHOG19-3 (Kipriynoav et al., 1998) for their anti-human CD16 counterparts (Arndt et al., Blood 94 (1999), 2562) using *Nco*I/*Hind*III and *Hind*III/*Xba*I restriction sites, respectively. The expression plasmid pKID19x16 containing dicistronic operon for cosecretion of two hybrid scFv was constructed by ligation of the *Bgl*II/*Xba*I restriction fragment from pHOG16-19 comprising the vector backbone and the *Bgl*II/*Xba*I fragment from pHOG19-16. CD19 x CD16 BsDb was produced in *E*. *coli* XL1 Blue (Stratagene, La Jolla, CA, USA) and isolated from bacterial periplasmic extract and culture medium by ammonium sulfate precipitation followed by IMAC, essentially as described for CD19 x CD3 BsDb (Kipriyanov et al., 1998). The final purification was achieved by ion-exchange FPLC on a Mono Q HR 5/5 column (Amersham Pharmacia, Freiburg, Germany) in 20 mM Tris-HCl, pH 8.5 with a linear 0-1 M NaCl gradient. The fractions containing BsDb were concentrated with simultaneous buffer exchange for PBS containing 50 mM imidazole, pH 7.0 using Ultrafree-15 centrifugal filter device (Millipore, Eschborn, Germany). Analysis of molecular forms of purified recombinant protein was performed by size-exclusion FPLC on a calibrated Superdex 200 HR 10/30 column (Amersham Pharmacia), as described previously (Kipriyanov et al., J. Mol. Biol. 293 (1999), 41).

### (C) Determination of diabody affinity by SPR

Kinetic constants of interaction of CD19 x CD16 BsDb with ECD of human FcγRIII were determined by SPR using BIAcore 2000 biosensor system (Biacore, Uppsala, Sweden). For immobilization on a streptavidin coated sensor chip SA (Biacore), the CD16 ECD was biotinylated according to a modified protocol of the ECL protein biotinylation module (Amersham Pharmacia). As a negative control, biotinylated porcine tubulin was used. The biotinylated Ags diluted in HBS-EP buffer (10 mM HEPES, 0.15 M NaCl, 3 mM EDTA, 0.005% polyoxyethylenesorbitan; Biacore) at a concentration of 10 µg/ml were applied to a sensor chip at a flow rate of 5 µl/min for 4 min resulting in immobilization of 800 resonance units (RU) of CD16 ECD and 900 RU of tubulin. All SPR measurements were carried out at a flow rate of 20 µl/min in HBS-EP at 25° C. Analyses were performed at 8 BsDb concentrations from 6.25 to 800 nM. Each injected sample (100 µl) was in contact with immobilized Ag for 5 min. The dissociation was followed for 10 min. After each cycle, the surface of the sensor chip was flushed with the buffer. Kinetic constants were calculated according to 1:1 (Langmuir) binding model using BIAevaluation version 3.0 software (Biacore).

### (D) Cell binding measurements

The human CD19⁺ B cell line JOK-1 and 293-CD16 cells were used for flow cytometry experiments performed as previously described (Kiriyanov et al., 1998). In brief, 5 x 10⁵ cells in 50 µl RPMI 1640 medium (GIBCO BRL, Eggenstein, Germany) supplemented with 10% FCS and 0.1% sodium azide (referred to as complete medium) were incubated with 100 µl of BsDb preparation for 45 min on ice. After washing with complete medium, the cells were incubated with 100 µl of 10 µg/ml anti-*c-myc* mAb 9E10 (DKFZ, Heidelberg, Germany) in the same buffer for 45 min on ice. After a second washing cycle, the cells were incubated with 100 µl of FITC-labeled goat anti-mouse IgG (GIBCO BRL, Karlsruhe, Germany) under the same conditions as before. The cells were then washed again and resuspended in 100 µl of 1 µg/ml solution of propidium iodide (Sigma-Aldrich, Taufkirchen, Germany) in complete medium to exclude dead cells. Fluorescence of stained cells was measured using a FACScan flow cytometer (Becton Dickinson, Mountain View, CA, USA). Mean fluorescence (F) was calculated using Cellquest software (Becton Dickinson) and the background fluorescence was subtracted. Equilibrium dissociation constants (*K*_{eq}) were determined as described by Benedict et al., J. Immunol. Methods 201 (1997), 223, by fitting the experimental values to the Lineweaver-Burk equation: 1/F = 1/Fₘₐₓ + (*K*_{eq}/Fₘₐₓ) (1/[BsDb]) using the software program GraphPad Prism (GraphPad Software, San Diego, CA).

### (E) In vitro cell surface retention

Cell surface retention assays were performed at 37°C under conditions preventing internalization of cell surface Ags, as described (Adams et al., Cancer Res. 58 (1998), 485) except that the detection of retained diabody was performed using anti-c-*myc* mAb 9E10 followed by FITC-labeled anti-mouse IgG. Kinetic dissociation constant (*k*_{off}) and half-life (*t*_{1/2}) values for dissociation of BsDb were deduced from a one-phase exponential decay fit of experimental data using GraphPad Prism (GraphPad Software).

### (F) Preparation of human effector cells

Human PBMCs were isolated from the blood of healthy donors by Ficoll (Sigma-Aldrich) density gradient centrifugation. For cytotoxicity assays in vitro, cultures of PBMC were grown overnight in RPMI 1640 (GIBCO BRL) supplemented with 10% heat inactivated FCS (GIBCO BRL), 2 mM glutamine, and recombinant human IL-2 (25 U/ml) (Eurocetus, Amsterdam, The Netherlands). For animal experiments, PBLs were preactivated in vitro by overnight incubation with immobilized mAb OKT3 (anti-human CD3), soluble mAb 15E8 (anti-human CD28), and recombinant human IL-2 (20 U/ml). The NK cells were negatively enriched from human PBMCs by immunomagnetic depletion of human T cells, B cells and myeloid cells using the NK cell isolation kit (Miltenyi Biotec, Bergisch Gladbach, Germany) to a purity of up to 90%, as estimated by FACS analysis, and were not additionally stimulated.

### (G) Cytotoxicity assays

The efficacy of the diabodies in mediating tumor cell lysis by human PBLs or NK cells was determined using the JAM test (Matzinger, J. Immunol. Meth. 145 (1991), 185). The CD19-expressing Burkitt's lymphoma cell line Raji was used as target cells. For the cell kill assay, 105 effector cells were mixed in round-bottomed microtiter plates with 10⁴ target cells labeled with [³H]thymidine in 100 µl medium plus 50 µl of diabody sample. After incubating the plate at 37°C, 5% CO₂ for 4 h, the cells were harvested and radioactivity was measured with a liquid scintillation beta counter (LKB, Wallach, Germany). Cytotoxicity related to the apoptosis-induced DNA fragmentation was calculated as % specific killing = *(S-E)*/*S* x 100, where *E* is experimentally retained labeled DNA in the presence of killers (in cpm) and *S* is retained DNA in the absence of killers (spontaneous). Synergistic effect of BsDbs in vitro was analyzed using PBLs from three healthy donors using four different E:T ratios. Each measurement was performed in triplicate. For each E:T ratio, the paired groups of results were compared by a paired *t*-test using GraphPad Prism (GraphPad Software).

### (H) Treatment of Burkitt's lymphoma in SCID mice

The SCID mice were obtained from Charles River (Sulzfeld, Germany) and kept under specific pathogen-free conditions at the Central Animal Facilities of the German Cancer Research Center. In each experiment, cohorts of five animals were used to permit accurate comparisons among differently treated groups. Mice were irradiated (300 rad) and received i.p. injections of 10 ml of anti-asialo-GM1 mAb (Wako, Neuss, Germany) according to the manufacturer's suggestions. One day later, 10⁷ Raji cells were injected s.c. dorsolaterally. Treatment was started after the tumors reached a size of 5 mm in diameter (day 0). At days 0, 7, and 15, the animals received i.v. injections of either PBS (control group) or 5 x 10⁶ preactivated human PBLs. Four h after each PBL injection, either PBS or antibody combinations were administered via the tail vein. These combinations included 50 µg CD19 x CD3 BsDb plus 25 µg mAb 15E8, or 50 µg CD19 x CD16 BsDb alone, or 25 µg CD19 x CD3 BsDb together with 25 µg CD19 x CD16 BsDb and 25 µg mAb 15E8. Tumor size was measured using a caliper every 2^{nd} day. Animals were followed until the s.c. tumors reached a maximal tolerated size of 15 mm in diameter and were killed by cervical dislocation. The days of sacrifice were recorded and were used for survival time analysis. The surviving animals were followed up to 60 days after the first treatment. For statistical evaluation, the follow-up duration of the tumor-treatment experiment was 30 days (end of experiment). The median survival times were estimated by the method described by Kaplan and Meier, J. Am. Statist. Assoc. 53 (1958), 457. Differences between survival curves were compared using a logrank test (Mantel and Haenszel, J. Natl. Cancer Inst. 22 (1959), 719).

### Example 2: Construction and production of CD19 x CD16 BsDb

To target human NK cells against malignant B cells, a small recombinant molecule with dual specificity for both the human B cell surface Ag CD19 and ECD of FcγRIII (CD16) was constructed. The scFv antibody fragments derived from hybridoma HD37 (Pezzutto et al., J. Immunol. 138 (1987), 2793) and A9 (Hombach et al., Int. J. Cancer 55 (1993), 830) were used to create CD19 x CD16 BsDb (Fig. 1). BsDb is a heterodimer formed by non-covalent association of two hybrid scFvs consisting of the V_{H} domain from one antibody connected by a short linker to the V_{L} domain of another antibody. *E*. *coli* cells containing the plasmid pKID19x16 for simultaneous expression of both components of the BsDb were grown and induced under conditions favoring their dimerization (Kipriyanov et al., J. Mol. Biol. 293 (1999), 41). Recombinant molecules were isolated by IMAC from crude periplasmic extracts and culture medium. Due to the higher expression of the V_{H}19-V_{L}16 hybrid scFv, the samples of IMAC purified heterodimeric diabody contained a significant amount of V_{H}19-V_{L}16 monomers and putative homodimers. The final separation of bispecific molecules was achieved by ion-exchange chromatography. Purified BsDb was mainly in a dimeric form with an *M*ᵣ around 60 kDa as demonstrated by gel filtration on a Superdex 200 column (Fig. 2A). In contrast, the non-functional V_{H}19-V_{L}16 molecules were mainly monomeric with an *M*ᵣ of 30 kDa (Fig. 2A). SDS-PAGE analysis demonstrated that the BsDb could be resolved into 2 protein bands corresponding to the calculated *M*ᵣ of 28,730 for V_{H}16-V_{L}19 scFv and *M*ᵣ of 29,460 for V_{H}19-V_{L}16 scFv (Fig. 2*B*). The expression vector SKID19x16 was deposited with the DSMZ (Deutsche Sammlung für Mikroorganismen und Zellen) according to the Budapest Treaty under DSM 14529 on September 24, 2001.

### Example 3: Ag-binding affinity

The association and dissociation rate constants for the anti-CD16 moiety of CD19 x CD16 BsDb were measured by SPR using biotinylated CD16 ECD as an Ag. BsDb exhibited a fairly high off-rate from CD16 coated sensor chip, thus making the regeneration of the biosensor surface unnecessary. The calculated off- and on-rate constants were 2.3 x 10⁻² s⁻¹ and 2.7 x 10⁴ s⁻¹ M⁻¹, respectively, resulting in a *K*_{d} of 8.5 x 10⁻⁷ M. A nearly identical affinity constant was deduced from the evaluation of steady-state binding levels (8.7 x 10⁻⁷ M).

**Table I**

| *Affinity and binding kinetics of CD19 x CD16 BsDb* | | | | | |
|---|---|---|---|---|---|
| Antigen | *k*ₒₙ (M⁻¹s⁻¹) | *k*_{off} (s⁻¹)^{a} | *t*_{1/2}(min)^{b} | *K*_{d} (M)^{c} | *K*_{eq}(M)^{d} |
| JOK-1 cells (CD19⁺) | n.d. | 1.1 x 10⁻³ | 10.6 | n.d. | 6.1 x 10⁻⁹ |
| 293-CD16 cells (CD16⁺) | n.d. | 3.2 x 10⁻³ | 3.6 | n.d. | 3.9 x 10⁻⁸ |
| CD16ECD | 2.7 x 10⁴ | 2.3 x 10⁻² | 0.5 | 8.5 x 10⁻⁷ | 8.7 x 10⁻⁷ |

| | | | | | |
|---|---|---|---|---|---|
| ^{a} Off-rate constants were either deduced from JOK-1 and 293-CD16 cell surface retention experiments or were measured together with on-rate constant (*k*ₒₙ) by SPR using immobilized biotinylated CD16 ECD. | | | | | |
| ^{b} The half-life values for dissociation of diabody-Ag complexes were deduced from the ratio ln2/*k*_{off}. | | | | | |
| ^{c} Affinity constants were calculated directly from the ratio *k*_{off}/*k*ₒₙ. | | | | | |
| ^{d} Equilibrium dissociation constants were either deduced from Lineweaver-Burk plots shown on Fig. 3*A* or from the steady-state analysis of SPR data. | | | | | |

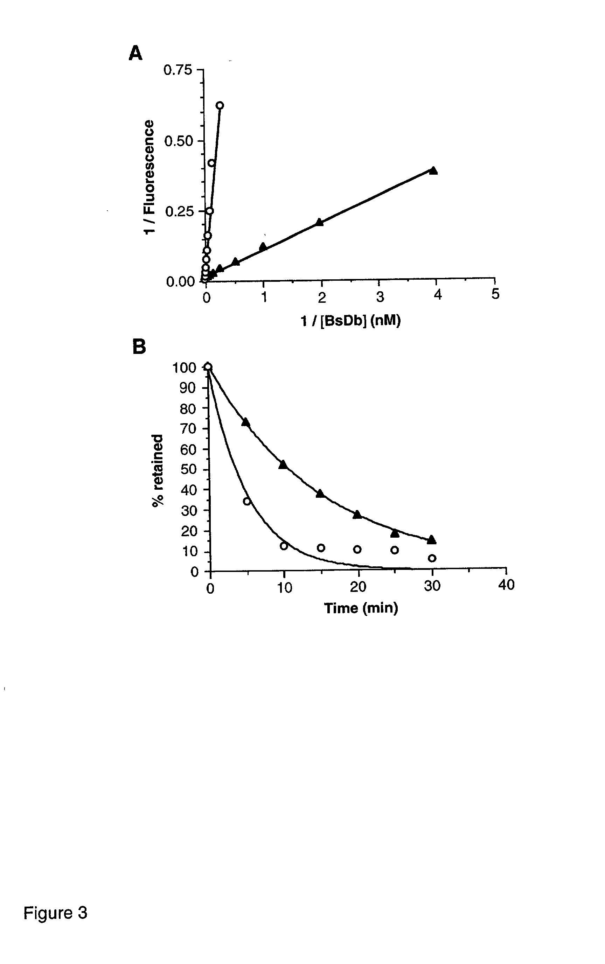

Since the CD16 target Ag could be present in many orientations on the BIAcore chip and some of its epitopes might be masked or destroyed due to biotinylation, the *K*_{d} determined by SPR may not accurately reflect the binding of BsDb to the surface of effector cells. Besides, the Ag-binding properties of the anti-CD19 moiety of the CD19 x CD16 BsDb could not be characterized by SPR because of the lack of free CD19. Therefore, the apparent equilibrium (*K*_{eq}) and off-rate (*k*_{off}) constants were also determined for binding to cell surface expressed CD19 and CD16 by flow cytometry. The flow cytometry experiments demonstrated a specific interaction of CD19 x CD16 BsDb with both CD19⁺ JOK-1 cells and 293-CD16 cells expressing ECD of human FcγRIII on their surface. The deduced *K*_{eq} value for binding to JOK-1 cells was 6.5-fold lower than for CD16-expressing cells (Fig. 3*A*). To investigate the biological relevance of the differences in direct binding experiments, the in vitro retention of the BsDb on the surface of both CD19⁺ and CD16⁺ cells at 37°C was determined by flow cytometry (Fig. 3*B*). CD19 x CD16 BsDb had a relatively short retention half-life (*t*_{1/2}) on 293-CD16 cells (3.6 min) and 3-fold longer *t*_{1/2} on the surface of CD19⁺ JOK-1 cells, thus correlating well with the lower CD16 binding affinity deduced from direct binding experiments. To determine whether the CD19 activity of BsDb is influenced by the second moiety of the bispecific molecule, CD19 x CD3 BsDb was used as a control in all flow cytometry experiments. Direct binding and cell surface retention on CD19⁺ JOK-1 cells were practically indistinguishable for both BsDbs. The calculated *K*_{eq} and *t*_{1/2} values were 5.7 nM and 10.8 min, respectively, for CD19 x CD3 BsDb, and 6.1 nM and 10.6 min for CD19 x CD16 BsDb. These results indicate that the second specificity present in the BsDb molecule does not significantly affect the affinity for binding to CD19.

### Example 4: Cytotoxicity in vitro

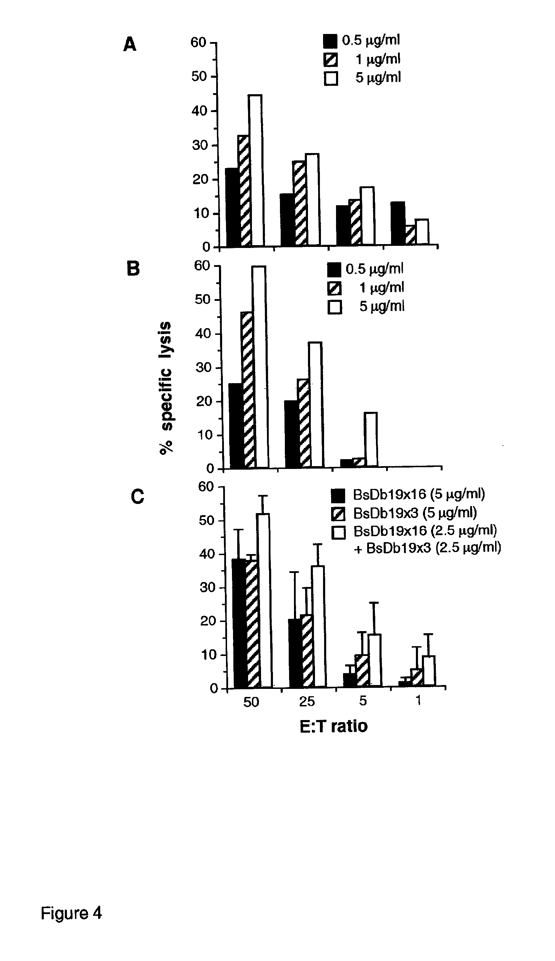

The ability of the CD19 x CD16 BsDb to induce tumor cell lysis by redirecting NK cell-mediated cytotoxicity was investigated using a JAM test, which is based on measuring DNA fragmentation in the target cell as a result of apoptosis (Matzinger, 1991). The death of CD19⁺ Raji cells in the presence of freshly prepared PBLs from a healthy donor was specifically triggered by CD19 x CD16 BsDb in a dose-dependent manner resulting in 45% of specific killing at a BsDb concentration of 5 µg/ml and E:T ratio of 50:1 (Fig. 4*A*). Substitution of PBLs by NK cells isolated from the blood of the same donor further increased the cytotoxic effect of CD19 x CD16 BsDb up to 60% under the same conditions (Fig. 4*B*). To examine the cytotoxic potential of different effector cell populations retargeted by BsDb, PBLs from three healthy donors in combination with either CD19 x CD16 BsDb or CD19 x CD3 BsDb, or both of them, were used. A higher tumor cell killing for each donor using a diabody combination could be observed than for any BsDb alone, although the absolute values of specific killing differed according to the donor. For example, at an E:T ratio of 25:1, the CD19 x CD16 BsDb alone, the CD19 x CD3 BsDb alone and a combination of both resulted in 2.1, 10.6, and 26.3% of specific killing for donor 1; 37.3, 30.8 and 39.4% for donor 2; and 20.2, 21.7 and 41.4% of specific killing for donor 3, respectively. For analyzing the results, a paired *t* test was used, which compares two paired groups and calculates the *t* ratio, *p* value and confidence interval based on the differences between each set of pairs. The results shown on Fig. 4*C* demonstrated that both BsDbs possessed fairly similar cytotoxic activities when used alone, and exhibited much higher activities when used together. It was no significant difference between the values of specific killing obtained using each BsDb alone (*p* = 0.1528). In contrast, the killing curve for the diabody combination differed significantly from those for CD19 x CD16 and CD19 x CD3 BsDb alone (*p* = 0.0068 and 0.0408, respectively).

### Example 5: Treatment of established Burkitt's lymphoma

To examine whether the synergistic effect of CD19 x CD16 BsDb and CD19 x CD3 BsDb could also be observed in vivo, a xenotransplant model of the Raji Burkitt's lymphoma in SCID mice was established. Raji cells after s.c. injection gave rise to locally growing tumors. Treatment was started when the tumors reached a size of 5 mm in diameter. At days 0, 7, and 15, cohorts of five mice received i.v. either PBS (control group) or in vitro preactivated human PBLs. Four h after each PBL inoculation, the mice were treated either with no antibody, or with CD19 x CD16 BsDb alone, or with CD19 x CD3 BsDb in combination with anti-human CD28 mAb 15E8 administered as a tail vein injection. The 5^{th} animal group received the combination of CD19 x CD16 BsDb, CD19 x CD3 BsDb and mAb 15E8. The total amount of injected BsDb was the same in all antibody treated groups, 50 µg (approx. 1 nmol) per animal. All animals in the control groups receiving PBS or PBLs alone did not show any tumor suppression and developed tumors larger than 1.5 cm in diameter in less than 3 weeks (Fig. 5). There was no significant difference between tumor growth in mice receiving PBS and mice receiving activated PBLs alone, which indicated that under the conditions used, any allogeneic reaction of the effector cells towards the tumor could be ignored. The animals were sacrificed when the tumors reached the maximum tolerated size of 15 mm in diameter. Sacrifice dates were recorded, and the median survival was calculated for each group (Fig. 6). The median survival times were not significantly different in the control groups receiving PBS and human PBLs alone at 21.5 and 23 days, respectively (*p* = 0.4469).

In contrast to control groups, all mice receiving a BsDb demonstrated significant tumor regression. The animals receiving three injections of CD19 x CD3 BsDb in combination with anti-CD28 mAb displayed a minimal tumor size on days 12-15, when 3 out of 5 mice were tumor-free. Afterwards, the tumors began to reappear and grew progressively in 2 animals (Fig. 5). One animal remained tumor-free until the end of monitoring (day 60 after the first treatment). Similar results were obtained for mice receiving CD19 x CD16 BsDb alone. In this group, all animals also demonstrated tumor regression till days 15-20, when 2 mice were tumor-free. Afterwards, however, the tumors started to grow again with comparable rates in all animals of this group (Fig. 5). The median survival times calculated for the groups receiving CD19 x CD3 BsDb plus mAb 15E8 and CD19 x CD16 BsDb alone were not significantly different, 33 and 32.5 days, respectively (*p* = 0.67), but were significantly different from the control groups (*p* < 0.01).

Survival was significantly improved in the group receiving the combination of CD19 x CD16 BsDb, CD19 x CD3 BsDb and anti-CD28 mAb, where 4 of 5 animals had no palpable tumors after the second injection (day 12, Fig. 5). These mice remained disease-free during the whole period of the experiment (30 days) and even 60 days after the first treatment. Compared with the other treatment groups this result was statistically significant (CD19 x CD3 BsDb plus mAb 15E8: *p* < 0.05; CD19 x CD16 BsDb: *p* < 0.01), and extremely significant in comparison with control groups (*p* < 0.001). These in vivo data clearly confirms the synergistic antitumor effect of a combinatory immunotherapeutic approach (using, e.g., CD19 x CD16 BsDb and CD19 x CD3 BsDb), which recruit different populations of human effector cells to the same tumor target.

## Claims

1. A combination of at least two antibodies, **characterized by** the following properties:
(a) it comprises at least two different multivalent antibodies, each one having at least two specificities and being **characterized by** features (b) and (d) or (b) and (c) as defined below;
(b) an antigen-binding domain specific to a tumor antigen;
(c) an antigen-binding domain specific to an antigen present on human T-cells; or
(d) an antigen-binding domain specific to an antigen present on CD3-epsilon negative human effector cells.

2. The combination according to claim 1, wherein the tumor antigen is human CD19.

3. The combination according to claim 1 or 2, wherein the CD19 antigen is expressed on human B-cells.

4. The combination according to claim 1, wherein the tumor antigen is human CD30.

5. The combination according to claim 4, wherein the CD30 antigen is expressed on human Hodgkin's cells.

6. The combination according to any one of claims 1 to 5, wherein the T cell antigen is CD3, CD28 or CD5.

7. The combination according to any one of claims 1 to 6, wherein the antigen present on CD3-epsilon negative human effector cells is CD16, CD64, CD32 or NKG-2D receptor.

8. The combination according to any one of claims 1 to 7, wherein the antibodies are devoid of constant regions.

9. The combination according to any one of claims 1 to 8, wherein at least two antibodies are multimeric antibodies.

10. The combination according to any one of claims 1 to 9, which comprises single chain Fv-antibodies comprising at least four immunoglobulin variable V_{H} and V_{L} domains, either separated by peptide linkers or by no linkers.

11. The combination according to any one of claims 1 to 9, which comprises heterodimers of two hybrid single chain Fv-antibodies, each consisting of V_{H} and V_{L} domains of different specificity against a tumor antigen and an antigen present on CD3-epsilon negative human effector cells or an antigen present on human T cells, either separated by peptide linkers or by no linkers.

12. The combination according to any one of claims 1 to 9, which comprises homodimers of single chain Fv-antibodies comprising at least four V_{H} and V_{L} domains of different specificity against a tumor antigen and an antigen present on CD3-epsilon negative human effector cells or an antigen present on human T cells, either separated by peptide linkers or by no linkers.

13. The combination of any one of claims 1 to 12, wherein said antigen-binding domains mimic or correspond to V_{H} and V_{L} regions from a natural antibody.

14. The combination according to claim 13, wherein said natural antibody is a monoclonal antibody, synthetic antibody, or humanized antibody.

15. The combination according to any one of claims 1 to 14, wherein at least one antibody is linked to an effector molecule having a conformation suitable for biological activity or selective binding to a solid support, a biologically active substance, a chemical agent, a peptide, a protein or a drug.

16. The combination according to any one of claims 1 to 15, comprising a third antibody having an antigen-binding domain as defined in claim 1(c) or (d) which is different from the antigen-binding domains of the first and second antibody.

17. The combination of any one of claims 1 to 15, comprising a first antibody which is a multivalent multimeric antibody specific to CD19 and CD16, a second antibody which is a multivalent multimeric antibody specific to CD19 and CD3, and, optionally, a third antibody which is specific to CD28.

18. Polynucleotides, which encode the antibodies of the combination according to any one of claims 1 to 17.

19. An expression vector comprising the polynucleotides of claim 18.

20. A host cell containing the expression vector of claim 19.

21. A process for the preparation of a combination of antibodies according to any one of claims 9 to 17, wherein (a) DNA sequences encoding the peptide linkers are ligated with the DNA sequences encoding the variable domains such that the peptide linkers connect the variable domains resulting in the formation of a DNA sequence encoding a monomer of a multivalent multimeric antibody, (b) the DNA sequences encoding the various monomers are expressed in a suitable expression system, and (c) the antibodies are combined.

22. A composition containing the combination of antibodies according to any one of claims 1 to 17, the polynucleotides of claim 18 or the expression vector of claim 19.

23. The composition of claim 22, which is a pharmaceutical composition optionally further comprising a pharmaceutically acceptable carrier or a diagnostic composition optionally further comprising suitable means for detection.

24. Use of the combination of antibodies of any of claims 1 to 17, the polynucleotides of claim 18 or the expression vector of claim 19 for the preparation of a pharmaceutical composition for treatment of B-cell malignancies, B-cell mediated autoimmune diseases or the depletion of B-cells.

25. Use according to claim 24, wherein said B-cell malignancy is non-Hodgkin's lymphoma.

26. Use of the combination of antibodies of any of claims 1 to 17, the polynucleotides of claim 18 or the expression vector of claim 19 for the preparation of a pharmaceutical composition for treatment of Hodgkin's disease.

27. Use of the polynucleotides of claim 18 or the expression vector of claim 19 for the preparation of a composition for gene therapy.
